# EUROPEAN PATENT SPECIFICATION

(11) **EP 3 928 780 B1**
(45) Date of publication and mention of the grant of the patent: **16.09.2026**
(21) Application number: 20759712.1
(22) Date of filing: 21.02.2020
(51) Int. Cl.: A61K 31/506, A61K 31/706, A61K 31/53, A61K 45/06, A61P 35/02

(54) **PHARMACEUTICAL COMPOSITION COMPRISING FLT3 INHIBITOR AND HYPOMETHYLATING AGENT FOR TREATING ACUTE MYELOID LEUKEMIA**
PHARMAZEUTISCHE ZUSAMMENSETZUNG MIT EINEM FLT3-INHIBITOR UND HYPOMETHYLIERUNGSMITTEL ZUR BEHANDLUNG VON AKUTER MYELOISCHER LEUKÄMIE
COMPOSITION PHARMACEUTIQUE COMPRENANT UN INHIBITEUR FLT3 ET UN AGENT HYPOMÉTHYLANT POUR LE TRAITEMENT DE LA LEUCÉMIE MYÉLOÏDE AIGUË

(30) Priority: 22.02.2019 KR 20190021228
(43) Date of publication of application: 29.12.2021
(73) Proprietor: Hanmi Pharm. Co., Ltd., Hwaseong-si, Gyeonggi-do 18536 (KR)
(72) Inventor: BAE, In Hwan, Hwaseong-si Gyeonggi-do 18469 (KR); KIM, Ji Sook, Hwaseong-si Gyeonggi-do 18469 (KR); CHOI, Jae Yul, Hwaseong-si Gyeonggi-do 18469 (KR); AHN, Young Gil, Hwaseong-si Gyeonggi-do 18469 (KR)
(74) Representative: Cooley (UK) LLP
(86) International application number: PCT/KR2020/002536
(87) International publication number: WO 2020/171646

(56) References cited:
- WO-A1-2018/139903
- WO-A1-2018/139903
- KR-A- 20180 124 055
- KR-A- 20180 124 055
- KR-B1- 101 954 370
- US-A1- 2011 183 975
- DAVER ET AL.: "TARGETING FLT3 MUTATIONS IN AML: REVIEW OF CURRENT KNOWLEDGE AND EVIDENCE", LEUKEMIA, vol. 33, 16 January 2019 (2019-01-16), pages 299 - 312, XP002807718
- HEIKO KONIG ET AL: "The combination of FLT3 inhibition and hypomethylation confers synergistic anti-leukemic effects on FLT3/ITD positive AML cell lines and primary cells", vol. 122, no. 21, 15 November 2013 (2013-11-15), pages 3965, XP009523247, ISSN: 0006-4971, Retrieved from the Internet <URL:https://ashpublications.org/blood/article/122/21/3965/70715/The-Combination-Of-FLT3-Inhibition-and> DOI: 10.1182/BLOOD.V122.21.3965.3965
- HEIKO KONIG, MARK LEVIS,: "The combination of FLT3 inhibition and hypomethylation confers synergistic anti-leukemic effects on FLT3/ITD positive AML cell lines and primary cells", BLOOD, vol. 122, no. 21, 15 November 2013 (2013-11-15), pages 3965, XP009523247, DOI: 10.1182/blood.V122.21.3965.3965

## Description

### TECHNICAL FIELD

The present disclosure relates to a pharmaceutical composition for treating acute myeloid leukemia, the pharmaceutical composition comprising a therapeutically effective combination of an Fms-like tyrosine kinase (Fms-like tyrosine kinase-3: FLT3) inhibitor and a hypomethylating agent, wherein the FLT3 inhibitor is 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine or a pharmaceutically acceptable salt, solvate, stereoisomer or tautomer thereof, and wherein the HMA is any one selected from 4-amino-1-β-D-ribofuranosyl- 1,3,5-triazin-2(1H)-one (azacitidine), 4-amino-1-((2R,4R,5R)-4-hydroxy-5- (hydroxymethyl)tetrahydrofuran-2-yl)-1,3,5-triazin-2(1H)-one (decitabine), and a pharmaceutically acceptable salt or hydrate thereof.

### BACKGROUND ART

Fms-like tyrosine kinase (Fms-like tyrosine kinase-3: FLT3) is one of the most frequently mutated genes in acute myeloid leukemia (AML). Mutant FLT3 refers to a mutant expressed in the leukemic cells of a subpopulation of AML patients. Activating mutations in FLT3, such as internal tandem duplication (ITD) in the juxtamembrane domain, occur in approximately 25% to approximately 30% of newly diagnosed AML cases (Patent Document 1). FLT3 mutations are known to occur in about one-third of patients with AML (Non-Patent Document 1).

There are several FLT3 inhibitors clinically applicable, but drug-resistant leukocytes were observed in AML patients treated with these FLT3 inhibitors and they showed drug resistance (Non-Patent Document 1). In addition, it is impossible to target AML stem/progenitor cells by a traditional AML standard chemotherapy, and thus recurrence is frequent in patients. Accordingly, there is a problem in that long-term efficacy is limited (Non-Patent Document 2). Therefore, there is a need for a method capable of effectively treating patients with mutant acute leukemia.

There has been an attempt to solve resistance to FLT3 inhibitors, in which administration of FLT3 inhibitors was studied by use of FLT3 inhibitors in combination with inhibitors of PI3K/Akt, MAPK and JAK/STAT signaling pathways (Non-Patent Document 3). Inhibitor of apoptosis protein (IAP) inhibitor refers to a protein that plays a role in mediating apoptosis, and these proteins are expressed in various ways in acute leukemia, and known to be involved in chemosensitivity, chemoresistance, disease progression, remission, and patient survival (Non-Patent Document 3). Use of IAP inhibitors in combination with FLT3 inhibitors has been studied for the treatment of AML and hematologic malignancies (Patent Document 2).

A hypomethylating agent (demethylating agent) refers to a drug that causes hypomethylation of DNA, and includes azacitidine, decitabine, idarubicin, etc. For example, decitabine is clinically used in primary and secondary myelodysplastic syndromes (MDS), etc. Azacitidine is a drug referred as the chemical name of "4-amino-1-β-D-ribofuranosyl-1,3,5-triazin-2(1H)-one" and is marketed under the trade name Vidaza. Azacitidine is known as a nucleoside metabolic inhibitor (hypomethylating agent) for the treatment of patients with FAB MDS subtypes.

### [Prior Art Documents]

[Patent Document 1] Korean Patent Publication No. 10-2018-0124055
[Patent Document 2] Korean Patent Publication No.10-2009-0087094
[Non-Patent Document 1] Mol Cancer Ther 2007;6(7). July 2007

[Non-Patent Document 2] J Natl Cancer Inst. Vol. 106, Issue 2, djt440, February 5, 2014
[Non-Patent Document 3] Oncogene. 2010 Sep 16;29(37):5120-34

Daver et al disclose, in LEUKEMIA, (2019), vol. 33, pages 299-312, "Targeting FLT3 mutations in AML: review of current knowledge and evidence".

Heiko Konig et al disclose, in BLOOD, (2013), vol. 122(21), page 3965, "The combination of FLT3 inhibition and hypomethylation confers synergistic anti-leukemic effects on FLT3/ITD positive AML cell lines and primary cells".

WO2018/139903A1 discloses a pyrimidine compound and pharmaceutical use thereof.

### DESCRIPTION OF EMBODIMENTS

### TECHNICAL PROBLEM

The present disclosure provides an alternative therapy for treating acute myeloid leukemia (AML) patients with mutant FLT3, leading to better therapeutic outcomes.

### SOLUTION TO PROBLEM

FLT3 is a promising therapeutic target for leukemia, and FLT3 mutation occurs in approximately 30% or more of acute myeloid leukemia (AML) patients. However, there is a growing interest in the development of drug resistance and refractories resulting from emergence of point mutations in targeted tyrosine kinases used for the treatment of AML. One approach to overcoming this resistance is identified by determining whether efficacy and therapeutic effects are enhanced by using structurally unrelated inhibitors and/or inhibitors of different signaling pathways in combination.

The invention is set out in the appended set of claims. The references to the methods of treatment by therapy or surgery or in vivo diagnosis methods along this disclosure of this description are to be interpreted as references to compounds, pharmaceutical compositions and medicaments of the present invention for use in those methods.

In accordance with claim 1, the present invention provides a pharmaceutical composition for use in treating acute myeloid leukemia (AML), the pharmaceutical composition comprising an Fms-like tyrosine kinase (Fms-like tyrosine kinase-3: FLT3) inhibitor or a pharmaceutically acceptable salt or solvate thereof, which is administered in combination with a hypomethylating agent (HMA) or a pharmaceutically acceptable salt or solvate thereof; wherein the FLT3 inhibitor is 5-chloro-N-(3-cyclopropyl-5-(((3R, 5S)-3,5-dimethylpiperazin-1-yl)methyl)phenyl)-4-(6-methyl-1H-indol-3-yl)pyrimidin-2-amine or a pharmaceutically acceptable salt, solvate, stereoisomer or tautomer thereof; and wherein the HMA is any one selected from 4-amino-1-β-D-ribofuranosyl-1,3,5-triazin-2(1H)-one (azacitidine), 4-amino-1-((2R,4R,5R)-4-hydroxy-5-(hydroxymethyl)tetrahydrofuran-2-yl)-1,3,5-triazin-2(1H)-one (decitabine), and a pharmaceutically acceptable salt or hydrate thereof. Preferred features are defined in the dependent claims.

### ADVANTAGEOUS EFFECTS OF DISCLOSURE

The present disclosure provides a pharmaceutical composition comprising an Fms-like tyrosine kinase (FLT3) inhibitor and a hypomethylating agent (HMA), wherein the FLT3 inhibitor is 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine or a pharmaceutically acceptable salt, solvate, stereoisomer or tautomer thereof, and wherein the HMA is any one selected from 4-amino-1-β-D-ribofuranosyl- 1,3,5-triazin-2(1H)-one (azacitidine), 4-amino-1-((2R,4R,5R)-4-hydroxy-5- (hydroxymethyl)tetrahydrofuran-2-yl)-1,3,5-triazin-2(1H)-one (decitabine), and a pharmaceutically acceptable salt or hydrate thereof.

The pharmaceutical composition of the present disclosure may provide enhanced therapeutic effects on AML patients with FLT3 mutant.

### BRIEF DESCRIPTION OF DRAWINGS

FIG. 1 shows inhibition of MOLM-13 cell growth by treatment with Compound A and azacitidine in combination, wherein the Y-axis represents a cell growth rate (%) and the X-axis represents a logarithmic concentration of Compound A (a logarithmic value of a unit concentration in nM);
FIG. 2 shows inhibition of cell growth by treatment with 2.5 nM of Compound A, 800 nM of azacitidine, or combination of 2.5 nM of Compound A and 800 nM of azacitidine, wherein the Y-axis represents a cell growth rate (%) and the X-axis represents each experimental group; and
FIG. 3 shows anti-tumor effects when nude mice xenografted with an MOLM-13-Luc2 cell line were treated with Compound A and azacitidine in combination, wherein the Y-axis represents a percentage (%) of surviving mice (survival rate) in each experimental group, and the X-axis represents the number of days of administration.

### MODE OF DISCLOSURE

Unless defined otherwise, all technical terms used herein have the same meanings as those generally understood by one of ordinary skill in the art to which the present disclosure belongs. Further, although methods or samples are described herein, those similar or equivalent thereto are also incorporated in the scope of the present disclosure. The numerical values described herein are considered to include the meaning of "about", unless otherwise specified.

The present disclosure provides a method, use, a combination, a kit, and a composition, each for the treatment of acute myeloid leukemia (AML), comprising an Fms-like tyrosine kinase (Fms-like tyrosine kinase-3: FLT3) inhibitor or a pharmaceutically acceptable salt or solvate thereof, and a hypomethylating agent (HMA) or a pharmaceutically acceptable salt or solvate thereof in combination effective for the treatment of AML.

In accordance with the present invention, there is provided a pharmaceutical composition for use in treating acute myeloid leukemia (AML), the pharmaceutical composition comprising an Fms-like tyrosine kinase (Fms-like tyrosine kinase-3: FLT3) inhibitor or a pharmaceutically acceptable salt or solvate thereof, which is administered in combination with a hypomethylating agent (HMA) or a pharmaceutically acceptable salt or solvate thereof; wherein the FLT3 inhibitor is 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine or a pharmaceutically acceptable salt, solvate, stereoisomer or tautomer thereof; and wherein the HMA is any one selected from 4-amino-1-β-D-ribofuranosyl-1,3,5-triazin-2(1H)-one (azacitidine), 4-amino-1-((2R,4R,5R)-4-hydroxy-5-(hydroxymethyl)tetrahydrofuran-2-yl)-1,3,5-triazin-2(1H)-one (decitabine), and a pharmaceutically acceptable salt or hydrate thereof.

As used herein, the AML includes AML with FLT3 mutation. In one specific embodiment, the AML includes mutant FLT3 polynucleotide-positive AML, FLT3 internal tandem duplication (ITD)-positive AML, or AML with FLT3 point mutations.

FLT3 is a member of the class III receptor tyrosine kinase (TK) family commonly expressed on the surface of hematopoietic stem cells. FLT3 and ligands thereof play an important role in proliferation, survival, and differentiation of pluripotent stem cells. FLT3 is expressed in many AML cases. Further, tyrosine kinase domain (TKD) mutations near D835 in activated FLT3 and activation loops with internal tandem duplication (ITD) in and around the juxtamembrane domain are found in 28% to 34% and 11% to 14% of AML cases, respectively. These activating mutations in FLT3 are tumorigenic and exhibit transforming activity in cells. Patients with FLT3-ITD mutation show poor prognosis in clinical studies, a higher relapse rate, a shorter duration of remission from initial therapy (6 months vs 11.5 months for those without FLT3-ITD mutations), as well as reduced disease-free survival (16% to 27% vs 41% at 5 years) and overall survival (OS) (15% to 31% vs 42% at 5 years). Recurrence after hematopoietic stem cell transplantation (HSCT) is also higher in FLT3-ITD patients (30% vs 16% for those without FLT3-ITD mutations at 2 years). Similar to the prognosis for first-line treatment, salvage chemotherapy on relapsed/refractory FLT3 mutationpositive AML patients shows a lower rate of remission, a shorter duration of remission to second-line relapse, and reduced OS, as compared to FLT3 mutation-negative patients.

In the pharmaceutical composition for use of the present invention, the FLT3 inhibitor is 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine or a pharmaceutically acceptable salt, solvate, stereoisomer or tautomer thereof.

Other FLT3 inhibitors include, but are not limited to, 4'-N-benzoyl staurosporine (ingredient name: midostaurin), 6-ethyl-3-[[3-methoxy-4-[4-(4-methyl-1-piperazinyl)-1-piperidinyl]phenyl]amino]-5-[(tetrahydro-2H-pyran-4-yl)amino]-2-pyrazinecarboxamide (ingredient name: gilteritinib), 1-(2-{5-[(3-methyloxetan-3-yl)methoxy]-1H-benzimidazol-1-yl}quinolin-8-yl)piperidin-4-amine (ingredient name: Crenolanib), 1-(5-(tert-butyl)isoxazol-3-yl)-3-(4-(7-(2-morpholinoethoxy)benzo[d]imidazo[2,1-b]thiazol-2-yl)phenyl)urea (ingredient name: quizartinib), 2-hydroxy-1-(2-((9-((1r,4r)-4-methylcyclohexyl)-9H- pyrido[4',3':4,5]pyrrolo[2,3-d] pyrimidin-2-yl)amino)-7,8-dihydro-1,6-naphthyridin-6(5H)-yl)ethanone (FLX925), (S,E)-N-(1-((5-(2-((4-cyanophenyl)amino)-4-(propylamino)pyrimidin-5-yl)pent-4-yn-1-yl)amino)-1-oxopropan-2-yl)-4-(dimethylamino)-N-methylbut-2-enamide (FF-10101), 6-[[(1R,2S)-2-aminocyclohexyl]amino]-7-fluoro-4-(1-methylpyrazol-4-yl)-1,2-dihydropyrrolo[3,4-c]pyridin-3-one (TAK-659), etc.; compounds having kinase inhibitory activity described in International Patent Application No. WO2018-139903 or compounds having FLT3 inhibitory activity described in Korean Patent Application No. 10-2018-0086768; or FLT3 inhibitors in the form of any pharmaceutically acceptable salt or hydrate thereof.

An example of an FLT3 inhibitor is a compound of Chemical Formula 1, a stereoisomer thereof, a tautomer thereof, or a combination thereof. 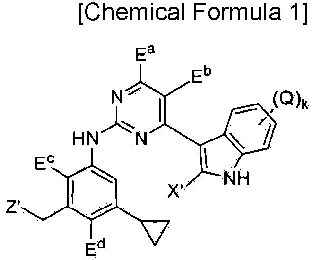 in Chemical Formula 1,
E^{a} is hydrogen, hydroxy, or C₁₋₄ alkoxy;
E^{b} is hydrogen, halogen, C₁₋₄ alkyl, or C₁₋₄ fluoroalkyl;
E^{c} and E^{d} are each independently hydrogen or hydroxy;
X' is hydrogen or hydroxy;
k is an integer of 1 to 2;
respective Q's are each independently hydroxy, halogen, C₁₋₄ alkyl, hydroxy C₁₋₄ alkyl, or C₁₋₄ alkoxy; and
Z' is a monovalent functional group represented by Chemical Formula 2; 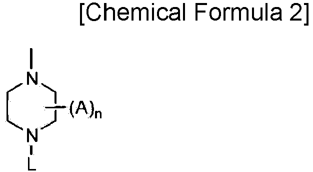
in Chemical Formula 2, n is an integer of 1 to 2;
respective A's are each independently a functional group selected from hydroxy, C₁₋₄ alkyl, and hydroxy C₁₋₄ alkyl, wherein at least one A is C₁₋₄ alkyl; and
L is hydrogen, C₁₋₄ alkyl, hydroxy, or hydroxy C₁₋₄ alkyl.

As used herein, the term "solvate" refers to a molecular complex of the compound of the present disclosure (or a pharmaceutically acceptable salt thereof) and one or more solvent molecules. Such solvent molecules are those known or commonly used in the pharmaceutical art, e.g., water, ethanol, etc. The term "hydrate" refers to a complex where the solvent molecule is water.

As used herein, the term "salt" or "pharmaceutically acceptable salt" refers to a pharmaceutically acceptable derivative of the disclosed compound, wherein a parent compound is modified by converting an existing acid or base moiety to a salt form thereof.

Another example of an FLT3 inhibitor is a compound of Chemical Formula 3, a stereoisomer thereof, a tautomer thereof, or a combination thereof. 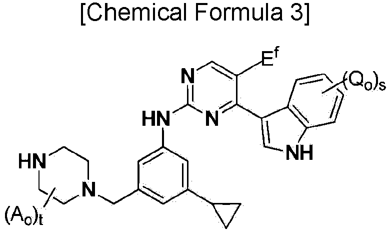 in Chemical Formula 3,
E^{f} is fluorine, chlorine, bromine, or iodine;
Qₒ is hydroxy, halogen, C₁₋₄ alkyl, hydroxy C₁₋₄ alkyl, or C₁₋₄ alkoxy;
s is an integer of 1 to 2;
Aₒ is a functional group selected from hydroxy, C₁₋₄ alkyl, and hydroxy C₁₋₄ alkyl; and t is an integer of 1 to 2.

Other FLT3 inhibitors include compounds having kinase inhibitory activity described in International Patent Application No. WO2018-139903, for example, compounds 1 to 55 in Table 1 below, or any pharmaceutically acceptable salts or hydrates thereof.

**[Table 1]**

| **No.** | **Compound name** |
|---|---|
| **1** | 2-(4-(3-((5-chloro-4-(6-fluoro-1*H*-indol-3-yl)pyrimidin-2-yl)amino)-5-cyclopropylphenyl)piperazin-1-yl)ethan-1-ol |
| **2** | 2-(4-(3-((5-chloro-4-(6-methyl-1H-indol-3-yl)pyrimidin-2yl)amino)-5-cyclopropylphenyl)piperazin-1-yl)ethan-1-ol |
| **3** | 5-chloro-*N*-(3-cyclopropyl-5-(4-(dimethylamino)piperidin-1-yl)phenyl)-4-(1*H-*indol-3-yl)pyrimidin-2-amine |
| **4** | (*S*)-1-((1-(3-((5-chloro-4-(1*H*-indol-3-yl)pyrimidin-2-yl)amino)-5-cyclopropylphenyl)piperidin-4-yl)(methyl)amino)propan-2-ol |
| **5** | (*S*)-1-((1-(3-((5-chloro-4-(6-methyl-1*H*-indol-3-yl)pyrimidin-2-yl)amino)-5-cyclopropylphenyl)piperidin-4-yl)(methyl)amino)propan-2-ol |
| **6** | 5-chloro-*N*-(3-cyclopropyl-5-(4-(dimethylamino)piperidin-1-yl)phenyl)-4-(6-methyl-1*H*-indol-3-yl)pyrimidin-2-amine |
| **7** | 2-(4-(3-((5-chloro-4-(6-methoxy-1*H*-indol-3-yl)pyrimidin-2-yl)amino)-5-cyclopropylphenyl)piperazin-1-yl)ethan-1-ol |
| **8** | (S)-1-(1-(3-((5-chloro-4-(1*H*-indol-3-yl)pyrimidin-2-yl)amino)-5-cyclopropylphenyl)piperidin-4-yl)pyrrolidin-3-ol |
| **9** | (*S*)-1-(1-(3-((5-chloro-4-(6-methyl-1*H*-indol-3-yl)pyrimidin-2-yl)amino)-5-cyclopropylphenyl)piperidin-4-yl)pyrrolidin-3-ol |
| **10** | 5-chloro-*N*-(3-cyclopropyl-5-(4-(dimethylamino)piperidin-1-yl)phenyl)-4-(6-methoxy-1*H*-indol-3-yl)pyrimidin-2-amine |
| **11** | (*S*)-1-(1-(3-((5-chloro-4-(6-methoxy-1*H*-indol-3-yl)pyrimidin-2-yl)amino)-5-cyclopropylphenyl)piperidin-4-yl)pyrrolidin-3-ol |
| **12** | 2-(4-(3-((4-(1*H*-indol-3-yl)-5-methylpyrimidin-2-yl)amino)-5-cyclopropylphenyl)piperazin-1-yl)ethan-1-ol |
| **13** | 5-chloro-*N*-(3-cyclopropyl-5-(4-morpholinopiperidin-1-yl)phenyl)-4-(6-methyl-1H-indol-3-yl)pyrimidin-2-amine |
| **14** | 5-chloro-*N*-(3-cyclopropyl-5-(4-(ethyl(methyl)amino)piperidin-1-yl)phenyl)-4-(6-methyl-1*H*-indol-3-yl)pyrimidin-2-amine |
| **15** | 5-chloro-*N*-(3-cyclopropyl-5-(4-(diethylamino)piperidin-1-yl)phenyl)-4-(6-methyl-1*H*-indol-3-yl)pyrimidin-2-amine |
| **16** | 5-chloro-*N*-(3-cyclopropyl-5-(3-(dimethylamino)pyrrolidin-1-yl)phenyl)-4-(6-methyl-1*H*-indol-3-yl)pyrimidin-2-amine |
| **17** | 2-(4-(3-((5-chloro-4-(6-methyl-1*H*-indol-3-yl)pyrimidin-2-yl)amino)-5-cyclopropylphenyl)piperazin-1-yl)-2-methylpropan-1-ol |
| **18** | *N*-(3-(4-aminopiperidin-1-yl)-5-cyclopropylphenyl)-5-chloro-4-(6-methyl-1*H-*indol-3-yl)pyrimidin-2-amine |
| **19** | 5-chloro-*N*-(3-cyclopropyl-5-(4-(methylamino)piperidin-1-yl)phenyl)-4-(6-methyl-1*H*-indol-3-yl)pyrimidin-2-amine |
| **20** | 2-(4-(3-((5-chloro-4-(6-fluoro-1*H*-indol-3-yl)pyrimidin-2-yl)amino)-5-cyclopropylphenyl)piperazin-1-yl)-2-methylpropan-1-ol |
| **21** | 2-(4-(3-((5-chloro-4-(6-methyl-1*H*-indol-3-yl)pyrimidin-2-yl)amino)-5-cyclopropylphenyl)piperidin-1-yl)ethan-1-ol |
| **22** | 2-(4-(3-((5-chloro-4-(6-chloro-1*H*-indol-3-yl)pyrimidin-2-yl)amino)-5-cyclopropylphenyl)piperazin-1-yl)ethan-1-ol |
| **23** | 5-chloro-*N*-(3-cyclopropyl-5-(4-(pyrrolidin-1-yl)piperidin-1-yl)phenyl)-4-(6-methyl-1*H*-indol-3-yl)pyrimidin-2-amine |
| **24** | 1-(1-(3-((5-chloro-4-(6-methyl-1*H*-indol-3-yl)pyrimidin-2-yl)amino)-5-cyclopropylphenyl)piperidin-4-yl)azetidin-3-ol |
| **25** | 2-(4-(3-((5-chloro-4-(1*H*-indol-3-yl)pyrimidin-2-yl)amino)-5-methoxyphenyl)piperazin-1-yl)ethan-1-ol |
| **26** | 2-(4-(3-((5-chloro-4-(6-fluoro-1*H*-indol-3-yl)pyrimidin-2-yl)amino)phenyl)piperazin-1-yl)ethan-1-ol |
| **27** | 2-(4-(3-((5-chloro-4-(1*H*-indol-3-yl)pyrimidin-2yl)amino)phenyl)piperidin-1-yl)ethan-1-ol |
| **28** | 2-(4-(3-((5-chloro-4-(1*H*-indol-3-yl)pyrimidin-2-yl)amino)phenyl) piperazin-1-yl)ethan-1-ol |
| **29** | 5-chloro-*N*-(3-(4-(dimethylamino)piperidin-1-yl)phenyl)-4-(1*H*-indol-3-yl)pyrimidin-2-amine |
| **30** | 5-chloro-*N*-(3-(3-(dimethylamino)pyrrolidin-1-yl)phenyl)-4-(1*H*-indol-3-yl)pyrimidin-2-amine |
| **31** | 2-(4-(3-((5-chloro-4-(6-fluoro-1*H*-indol-3-yl)pyrimidin-2-yl)amino)-5-methoxyphenyl)piperazin-1-yl)ethan-1-ol |
| **32** | 2-(4-(3-((5-chloro-4-(1*H*-indol-3-yl)pyrimidin-2-yl)amino)-5-isopropoxyphenyl)piperazin-1-yl)ethan-1-ol |
| **33** | 2-(4-(3-((5-chloro-4-(6-fluoro-1*H*-indol-3-yl)pyrimidin-2-yl)amino)-5-isopropoxyphenyl)piperazin-1-yl)ethan-1-ol |
| **34** | 5-chloro-*N*-(3-cyclopropyl-5-(piperazin-1-ylmethyl)phenyl)-4-(6-fluoro-1*H*-indol-3-yl)pyrimidin-2-amine |
| **35** | 2-(4-(3-((5-chloro-4-(6-fluoro-1*H*-indol-3-yl)pyrimidin-2-yl)amino)-5-methoxybenzyl)piperazin-1-yl)ethan-1-ol |
| **36** | 2-(4-(3-((5-chloro-4-(6-fluoro-1*H*-indol-3-yl)pyrimidin-2-yl)amino)benzyl)piperazin-1-yl)ethan-1-ol |
| **37** | 2-(4-(3-((5-chloro-4-(1*H*-indol-3-yl)pyrimidin-2-yl)amino)-5-methoxybenzyl)piperazin-1-yl)ethan-1-ol |
| **38** | 2-(4-(3-((5-chloro-4-(1*H*-indol-3-yl)pyrimidin-2-yl)amino)-5-cyclopropylbenzyl)piperazin-1-yl)-2-methylpropan-1-ol |
| **39** | (S)-1-((1-(3-((5-chloro-4-(1*H*-indol-3-yl)pyrimidin-2-yl)amino)-5-cyclopropylbenzyl)piperidin-4-yl)(methyl)amino)propan-2-ol |
| **40** | (*S*)-1-((1-(3-((5-chloro-4-(6-methyl-1*H*-indol-3-yl)pyrimidin-2-yl)amino)-5-cyclopropylbenzyl)piperidin-4-yl)(methyl)amino)propan-2-ol |
| **41** | 2-(4-(3-((5-chloro-4-(6-methyl-1*H*-indol-3-yl)pyrimidin-2-yl)amino)-5-cyclopropylbenzyl)piperazin-1-yl)-2-methylpropan-1-ol |
| **42** | (*S*)-1-(1-(3-((5-chloro-4-(1*H*-indol-3-yl)pyrimidin-2-yl)amino)-5-cyclopropylbenzyl)piperidin-4-yl)pyrrolidin-3-ol |
| **43** | (*S*)-1-(1-(3-((5-chloro-4-(6-methyl-1*H*-indol-3-yl)pyrimidin-2-yl)amino)-5-cyclopropylbenzyl)piperidin-4-yl)pyrrolidin-3-ol |
| **44** | (*S*)-1-(1-(3-((5-chloro-4-(6-methoxy-1*H*-indol-3-yl)pyrimidin-2-yl)amino)-5-cyclopropylbenzyl)piperidin-4-yl)pyrrolidin-3-ol |
| **45** | 1-(3-((5-chloro-4-(6-methyl-1*H*-indol-3-yl)pyrimidin-2-yl)amino)-5-cyclopropylbenzyl)piperidin-4-ol |
| **46** | (*S*)-5-chloro-*N*-(3-cyclopropyl-5-((3-(dimethylamino)pyrrolidin-1-yl)methyl)phenyl)-4-(6-methyl-1*H*-indol-3-yl)pyrimidin-2-amine |
| **47** | 1-(4-(3-((5-chloro-4-(6-fluoro-1*H*-indol-3-yl)pyrimidin-2-yl)amino)-5-cyclopropylbenzyl)piperazin-1-yl)-2-hydroxyethan-1-one |
| **48** | 1-(4-(3-((5-chloro-4-(6-methyl-1*H*-indol-3-yl)pyrimidin-2-yl)amino)-5-cyclopropylbenzyl)piperazin-1-yl)-2-hydroxyethan-1-one |
| **49** | 2-(4-(3-((5-chloro-4-(6-ethyl-1*H*-indol-3-yl)pyrimidin-2-yl)amino)-5-cyclopropylbenzyl)piperazin-1-yl)ethan-1-ol |
| **50** | (3-((5-chloro-4-(1*H*-indol-3-yl)pyrimidin-2-yl)amino)-5-methoxyphenyl)(4-(2-hydroxyethyl)piperazin-1-yl)methanone |
| **51** | 1-(2-(3-((5-chloro-4-(6-methyl-1*H*-indol-3-yl)pyrimidin-2-yl)amino)-5-cyclopropylphenoxy)ethyl)piperidin-4-ol |
| **52** | 1-(2-(3-((5-chloro-4-(6-methyl-1*H*-indol-3-yl)pyrimidin-2-yl)amino-5-ethylphenoxy)ethyl)piperidin-4-ol |
| **53** | (*R*)-2-(3-(3-((5-chloro-4-(6-methyl-1*H*-indol-3-yl)pyrimidin-2-yl)amino)-5-cyclopropylphenoxy)pyrrolidin-1-yl)ethan-1-ol |
| **54** | 2-(4-(3-((5-chloro-4-(6-methyl-1*H*-indol-3-yl)pyrimidin-2-yl)amino)-5-cyclopropylphenoxy)piperidin-1-yl)ethan-1-ol |
| **55** | 2-(4-(3-((5-chloro-4-(1*H*-indol-3-yl)pyrimidin-2-yl)amino)-5-methoxyphenoxy)piperidin-1-yl)ethan-1-ol |

Other FLT3 inhibitors include compounds having FLT3 inhibitory activity described in Korean Patent Application No. 10-2018-0086768, for example, compounds 1 to 32 in Table 2 below, or any pharmaceutically acceptable salts or hydrates thereof.

**[Table 2]**

| **No.** | **Compound name** |
|---|---|
| **1** | 5-chloro-*N*-(3-cyclopropyl-5-(((3*R*, 5*S*)-3,5-dimethylpiperazin-1-yl)methyl)phenyl)-4-(6-fluoro-1*H*-indol-3-yl)pyrimidin-2-amine |
| **2** | 5-chloro-4-(6-chloro-1*H*-indol-3-yl)-*N*-(3-cyclopropyl-5-(((3*R*, 5*S*)-3,5-dimethylpiperazin-1-yl)methyl)phenyl)pyrimidin-2-amine |
| **3** | 2-((2*R*, 6*S*)-4-(3-((5-chloro-4-(6-fluoro-1*H*-indol-3-yl)pyrimidin-2-yl)amino)-5-cyclopropylbenzyl)-2,6-dimethylpiperazin-1-yl)ethan-1-ol |
| **4** | 2-((2*R*, 6*S*)-4-(3-((5-chloro-4-(1*H*-indol-3-yl)pyrimidin-2-yl)amino)-5-cyclopropylbenzyl)-2,6-dimethylpiperazin-1-yl)ethan-1-ol |
| **5** | 2-((2*R*, 6*S*)-4-(3-((5-chloro-4-(6-methyl-1*H*-indol-3-yl)pyrimidin-2-yl)amino)-5-cyclopropylbenzyl)-2,6-dimethylpiperazin-1-yl)ethan-1-ol |
| **6** | (*R*)-5-chloro-*N-*(3-cyclopropyl-5-((3-methylpiperazin-1-yl)methyl)phenyl)-4-(1*H-*indol-3-yl)pyrimidin-2-amine |
| **7** | (*R*)-5-chloro-*N*-(3-cyclopropyl-5-((3-methylpiperazin-1-yl)methyl)phenyl)-4-(6-methyl-1*H*-indol-3-yl)pyrimidin-2-amine |
| **8** | 5-chloro-*N*-(3-cyclopropyl-5-(((3*R*, 5*S*)-3,5-dimethylpiperazin-1-yl)methyl)phenyl)-4-(6-methyl-1*H*-indol-3-yl)pyrimidin-2-amine |
| **9** | 5-chloro-*N*-(3-cyclopropyl-5-(((3*S*, 5*R*)-3-ethyl-5-methylpiperazin-1-yl)methyl)phenyl)-4-(6-methyl-1*H*-indol-3-yl)pyrimidin-2-amine |
| **10** | 5-chloro-*N*-(3-cyclopropyl-5-((3,5-dimethylpiperazin-1-yl)methyl)phenyl)-4-(6-methyl-1*H*-indol-3-yl)pyrimidin-2-amine |
| **11** | *N*-(3-cyclopropyl-5-(((3*R*,5*S*)-3,5-dimethylpiperazin-1-yl)methyl)phenyl)-4-(6-methyl-1*H*-indol-3-yl)pyrimidin-2-amine |
| **12** | *N*-(3-cyclopropyl-5-(((3*R*, 5*S*)-3,5-dimethylpiperazin-1-yl)methyl)phenyl)-5-fluoro-4-(6-methyl-1*H*-indol-3-yl)pyrimidin-2-amine |
| **13** | *N*-(3-cyclopropyl-5-(((3R, 5*S*)-3,5-dimethylpiperazin-1-yl)methyl)phenyl)-4-(1*H-*indol-3-yl)-5-methylpyrimidin-2-amine |
| **14** | *N*-(3-cyclopropyl-5-(((3*R*, 5*S*)-3,5-dimethylpiperazin-1-yl)methyl)phenyl)-5-methyl-4-(6-methyl-1*H*-indol-3-yl)pyrimidin-2-amine |
| **15** | *N*-(3-cyclopropyl-5-(((3*R*, 5*S*)-3,5-dimethylpiperazin-1-yl)methyl)phenyl)-4-(6-methyl-1*H*-indol-3-yl)-5-(trifluoromethyl)pyrimidin-2-amine |
| **16** | (3-(5-chloro-2-((3-cyclopropyl-5-(((3*R*, 5*S*)-3,5-dimethylpiperazin-1-yl)methyl)phenyl)amino)pyrimidin-4-yl)-1*H*-indol-6-yl)methanol |
| **17** | 5-chloro-*N*-(3-cyclopropyl-5-(((3*R*, 5*S*)-3,5-dimethylpiperazin-1-yl)methyl)phenyl)-4-(5-methoxy-6-methyl-1*H*-indol-3-yl)pyrimidin-2-amine |
| **18** | 3-(5-chloro-2-((3-cyclopropyl-5-(((3*R*, 5*S*)-3,5-dimethylpiperazin-1-yl)methyl)phenyl)amino)pyrimidin-4-yl)-6-methyl-1*H*-indol-5-ol |
| **19** | 3-(5-chloro-2-((3-cyclopropyl-5-(((3*R*, 5*S*)-3,5-dimethylpiperazin-1-yl)methyl)phenyl)amino)pyrimidin-4-yl)-6-methylindolin-2-one |
| **20** | 5-chloro-*N*-(3-cyclopropyl-5-(((3*R*, 5*S*)-3,5-dimethylpiperazin-1-yl)methyl)phenyl)-4-methoxy-6-(6-methyl-1*H*-indol-3-yl)pyrimidin-2-amine |
| **21** | 5-chloro-2-((3-cyclopropyl-5-(((3*R*, 5*S*)-3,5-dimethylpiperazin-1-yl)methyl)phenyl)amino)-6-(6-methyl-1*H*-indol-3-yl)pyrimidin-4-ol |
| **22** | 3-(5-chloro-2-((3-cyclopropyl-5-(((3*R*, 5*S*)-3,5-dimethylpiperazin-1-yl)methyl)phenyl)amino)pyrimidin-4-yl)-6-methyl-1*H*-indol-7-ol |
| **23** | 2-((5-chloro-4-(6-methyl-1*H*-indol-3-yl)pyrimidin-2-yl)amino)-4-cyclopropyl-6-(((3*R*, 5*S*)-3,5-dimethylpiperazin-1-yl)methyl)phenol |
| **24** | 4-((5-chloro-4-(6-methyl-1*H*-indol-3-yl)pyrimidin-2-yl)amino)-2-cyclopropyl-6-(((3*R*, 5*S*)-3,5-dimethylpiperazin-1-yl)methyl)phenol |
| **25** | (*R*)-5-chloro-*N*-(3-cyclopropyl-5-((3,3,5-trimethylpiperazin-1-yl)methyl)phenyl)-4-(6-methyl-1*H*-indol-3-yl)pyrimidin-2-amine |
| **26** | ((2*R*, 6*R*)-4-(3-((5-chloro-4-(6-methyl-1*H*-indol-3-yl)pyrimidin-2-yl)amino)-5-cyclopropylbenzyl)-6-methylpiperazin-2-yl)methanol |
| **27** | (*R*)-5-chloro-*N*-(3-cyclopropyl-5-((6-methyl-4,7-diazaspiro[2.5]octan-7-yl)methyl)phenyl)-4-(6-methyl-1*H*-indol-3-yl)pyrimidin-2-amine |
| **28** | 5-chloro-*N*-(3-cyclopropyl-5-(((3*R*, 5*R*)-3,5-dimethylpiperazin-1-yl)methyl)phenyl)-4-(6-methyl-1*H*-indol-3-yl)pyrimidin-2-amine |
| **29** | 5-chloro-*N*-(3-cyclopropyl-5-(((3*S*, 5*S*)-3,5-dimethylpiperazin-1-yl)methyl)phenyl)-4-(6-methyl-1*H*-indol-3-yl)pyrimidin-2-amine |
| **30** | 5-chloro-*N*-(3-cyclopropyl-5-(((3R, 5*S*)-3,4,5-trimethylpiperazin-1-yl)methyl)phenyl)-4-(6-methyl-1*H*-indol-3-yl)pyrimidin-2-amine |
| **31** | (2*R*, 6*S*)-4-(3-((5-chloro-4-(6-methyl-1*H*-indol-3-yl)pyrimidin-2-yl)amino)-5-cyclopropylbenzyl)-2,6-dimethylpiperazin-1-ol |
| **32** | (2*R*, 6*S*)-4-(3-cyclopropyl-5-((4-(6-methyl-1*H*-indol-3-yl)pyrimidin-2-yl)amino)benzyl)-2,6-dimethylpiperazin-1-ol |

In the pharmaceutical composition for use according to the present invention, the FLT3 inhibitor is 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine, or a pharmaceutically acceptable salt, solvate, stereoisomer or tautomer thereof. In some embodiments, the FLT3 inhibitor is 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine, or a pharmaceutically acceptable salt or hydrate thereof.

As used herein, a hypomethylating agent (HMA) refers to a substance for DNA hypomethylation or a DNA demethylating agent. DNA methylation is a major mechanism regulating gene expression in cells, and when DNA methylation is increased, activity of suppressor genes that regulate cell division and proliferation is blocked, and as a result, cell division is not controlled and cancer progresses. The hypomethylating agent may function, for example, as an antimetabolic agent that interferes with DNA methylation and restores tumor suppressor genes to regulate tumor growth, or has a structure similar to a substance required for tumor cell metabolism to interfere with cellular metabolism and to exhibit a tumor growth inhibitory action.

In the pharmaceutical composition for use according to the present invention, the HMA is 4-amino-1-β-D-ribofuranosyl-1,3,5-triazin-2(1H)-one (ingredient name: azacitidine), 4-amino-1-((2R,4R,5R)-4-hydroxy-5-(hydroxymethyl)tetrahydrofuran-2-yl)-1,3,5-triazin-2(1H)-one (ingredient name: decitabine), or a pharmaceutically acceptable salt or hydrate thereof.

In one specific embodiment, the HMA may be 4-amino-1-β-D-ribofuranosyl-1,3,5-triazin-2(1H)-one, or 4-amino-1-((2R,4R,5R)-4-hydroxy-5-(hydroxymethyl)tetrahydrofuran-2-yl)-1,3,5-triazin-2(1H)-one, and the FLT3 inhibitor may be 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine.

In one specific embodiment, the HMA may be 4-amino-1-β-D-ribofuranosyl-1,3,5-triazin-2(1H)-one, and the FLT3 inhibitor may be 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine.

In one specific embodiment, the HMA may be 4-amino-1-((2R,4R,5R)-4-hydroxy-5-(hydroxymethyl)tetrahydrofuran-2-yl)-1,3,5-triazin-2(1H)-one, and the FLT3 inhibitor may be 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine.

5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine as the FLT3 inhibitor according to one specific embodiment inhibits kinases, such as SYK, which are known to be associated with AML resistance. Among them, SYK kinase transactivates FLT3 by a direct physical interaction, is critical for the development of FLT3-ITD-induced myeloid neoplasia, and is more highly activated in primary FLT3-ITD-positive AML. Therefore, activation of other signaling pathways of kinases such as SYK may contribute to resistance in the treatment of AML patients.

The FLT3 inhibitor according to one specific embodiment has excellent therapeutic effects on AML with FLT3 mutation, which has a high risk of recurrence after treatment, poor prognosis, and a reduction in overall survival.

The FLT3 inhibitor according to one specific embodiment exhibits clinical benefits in AML patients with resistance to traditional therapeutic agents. In about 30% of AMK patients, activating mutations in ITD of FLT3 and point mutations in TKD are reported to be oncogenic driver mutation. For example, the mutation of TKD may further comprise ITD.

In one specific embodiment, AML which is a therapeutic target of the pharmaceutical composition may be AML with FLT3 mutation.

The AML may have a mutation in TKD of an amino acid sequence of FLT3 (FLT3-TKD). The FLT3-TKD mutation may further comprise ITD. The FLT3-TKD mutation may comprise any one selected from FLT3(D835Y), FLT3(F691L), FLT3(F691L/D835Y), FLT3(ITD/D835Y), FLT3(ITD/F691L), and combinations thereof.

The pharmaceutical composition according to one specific embodiment, in which the FLT3 inhibitor or a pharmaceutically acceptable salt or solvate thereof, and the HMA or a pharmaceutically acceptable salt or solvate thereof are administered in combination, may have excellent therapeutic effects on AML with FLT3 mutations.

In one specific embodiment, the AML may be mutant FLT3 polynucleotide-positive AML, FLT3 ITD-positive AML, or AML with FLT3 point mutations.

In one specific embodiment, the AML may have mutations in TKD of the amino acid sequence of FLT3 (FLT3-TKD).

In one specific embodiment, the FLT3-TKD mutation may further comprise ITD.

In one specific embodiment, the FLT3-TKD mutation may comprise any one selected from FLT3(D835Y), FLT3(F691L), FLT3(F691L/D835Y), FLT3(ITD/D835Y), FLT3(ITD/F691L), and combinations thereof.

In one specific embodiment, the FLT3 inhibitor may be 5-chloro-N-(3-cyclopropyl-5-(((3R, 5S)-3,5-dimethylpiperazin-1-yl)methyl)phenyl)-4-(6-methyl-1H-indol-3-yl)pyrimidin-2-amine, or a pharmaceutically acceptable salt or hydrate thereof.

The pharmaceutical composition according to a specific embodiment may comprise the FLT3 inhibitor, or any pharmaceutically acceptable salt or hydrate thereof for treating AML with FLT3 mutation, for example, AML with FLT3 ITD and TKD point mutations, e.g., FLT3(ITD/D835Y) and FLT3(ITD/F691L) mutations.

The FLT3-TKD mutation may comprise one amino acid mutation or a plurality of amino acid mutations at positions 823 to 861 of the FLT3 amino acid sequence. The TKD mutation may comprise a mutation of at least one amino acid selected from the group consisting of amino acids at positions 835, 836, and 842 of the FLT3 amino acid sequence. For example, the TKD mutation may comprise a mutation of an amino acid at position 835 of the FLT3 amino acid sequence. For example, the TKD mutation may include substitution of valine, tyrosine, histidine, glutamic acid, or asparagine for aspartic acid at position 835 of the FLT3 amino acid sequence. For example, the TKD mutation may include substitution of leucine or aspartic acid for isoleucine at position 836 of the FLT3 amino acid sequence. For example, the TKD mutation may include substitution of cysteine or histidine for tyrosine at position 842 of the FLT3 amino acid sequence. For example, the mutation may be FLT3(D835Y).

The FLT3-TKD mutation may have a mutation of at least one amino acid selected from the group consisting of amino acids at positions 621, 627, 676, 691, and 697 of the FLT3 amino acid sequence. For example, the TKD mutation may have substitution of leucine for phenylalanine at position 691 of the FLT3 amino acid sequence. For example, the mutation may be FLT3(F691L).

The TKD mutation may further include ITD. For example, the mutation may be FLT3(ITD/D835Y) or FLT3(ITD/F691L).

5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine as the FLT3 inhibitor according to one specific embodiment overcomes resistance caused by FLT3 mutations and shows therapeutic effects in an *in vivo* study using Ba/F3 cells expressed in an FLT3 ITD/F691L or FLT3 ITD/D835Y xenografted mouse model.

The FLT3 inhibitor according to one specific embodiment exhibits the effect capable of overcoming resistance of AML therapy. For example, the FLT3 inhibitor exhibits inhibitory activity against drug-resistant point mutants (D835Y, F691L, or F691LID835Y) of FLT3 due to acquired D835Y and F691L point mutations in FLT3-TKD. In one specific embodiment, the TKD mutation may have substitution of tyrosine for aspartic acid at position 835 of the FLT3 amino acid sequence. In one specific embodiment, the mutation may be FLT3(D835Y) or FLT3(ITD/D835Y). In one specific embodiment, the TKD mutation may have substitution of leucine for phenylalanine at position 691 of the FLT3 amino acid sequence. The mutation may be FLT3(F691L) or FLT3(ITD/F691L).

As a result of an *in vitro* site-directed competition binding assay using AML resistance cell line, the FLT3 inhibitor according to one specific embodiment overcomes resistance due to FLT3 mutations and shows a therapeutic effect, demonstrated through a standard proliferation assay, immunoblotting, and apoptosis analysis.

The FLT3 inhibitor according to one specific embodiment strongly inhibits FLT3(ITD/D835Y) and FLT3(ITD/F691L) mutations in a preclinical study. The FLT3 inhibitor according to one specific embodiment exhibits high *in vitro* binding affinity to both the two mutations, and exhibits strong inhibitory activity *in vitro* and *in vivo* against a Ba/F3 cell line expressing FLT3(ITD/D835Y) or FLT3(ITD/F691L). Furthermore, the FLT3 inhibitor according to one specific embodiment exhibits high cytotoxicity in an MOLM-14 cell line harboring FLT3 ITD and overcomes FL-induced drug resistance. The FLT3 inhibitor according to one specific embodiment may strongly inhibit phosphorylation of SYK, STAT3, and STAT5 in KG-la cells.

Further, the FLT3 inhibitor according to one specific embodiment may exhibit a synergistic effect, when used in combination with another one or more therapeutic agents for leukemia, e.g., inhibitor of apoptosis protein (IAP) inhibitors or chemotherapy.

The FLT3 inhibitor or a pharmaceutically acceptable salt or solvate thereof, and HMA or a pharmaceutically acceptable salt or solvate thereof may be administered simultaneously, sequentially, in reverse order, or individually.

Administration routes include, but are not limited to, oral, intravenous, intraarterial, intraperitoneal, intradermal, transdermal, intrathecal, intramuscular, intranasal, transmucosal, subcutaneous and rectal administration.

Formulations for administration according to one specific embodiment may be used after being formulated according to common methods in any appropriate formulation, including oral preparations such as tablets, powders, granules, capsules, suspensions, emulsions, syrups, aerosols, etc., external preparations such as ointments, creams, etc., injections, suppositories, and sterile injectable solutions, etc.

As the FLT3 inhibitor according to one specific embodiment, 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine, or any pharmaceutically acceptable salt or hydrate thereof may be orally administered.

As the HMA according to one specific embodiment, 4-amino-1-β-D-ribofuranosyl-1,3,5-triazin-2(1H)-one (ingredient name: azacitidine) may be administered intravenously, intraperitoneally, or subcutaneously.

As the HMA according to one specific embodiment, 4-amino-1-((2R,4R,5R)-4-hydroxy-5-(hydroxymethyl)tetrahydrofuran-2-yl)-1,3,5-triazin-2(1H)-one (ingredient name: decitabine) may be administered intravenously, intraperitoneally, or subcutaneously.

In one specific embodiment, the FLT3 inhibitor or a pharmaceutically acceptable salt or solvate thereof, and the HMA or a pharmaceutically acceptable salt or solvate thereof may be comprised in a therapeutically effective amount, respectively.

In one specific embodiment, the FLT3 inhibitor or a pharmaceutically acceptable salt or solvate thereof, and the HMA or a pharmaceutically acceptable salt or solvate thereof may be comprised as a therapeutic agent for AML in a therapeutically effective amount to be administered simultaneously, sequentially, in reverse order, or individually.

The FLT3 inhibitor in the pharmaceutical composition according to a specific embodiment may be administered in an amount of 0.01 mg to 3000 mg, e.g., 0.1 mg to 1000 mg or 6 mg to 600 mg. Alternatively, the FLT3 inhibitor may be administered in an amount of 0.001 mg/kg of body weight to 200 mg/kg of body weight, e.g., 0.05 mg/kg of body weight to 100 mg/kg of body weight, 0.1 mg/kg of body weight to 50 mg/kg of body weight, or 0.1 mg/kg of body weight to 10 mg/kg of body weight. Alternatively, the FLT3 inhibitor may be administered in a daily dose of 0.001 mg/kg of body weight to 200 mg/kg of body weight, e.g., in a daily dose of 0.05 mg/kg of body weight to 50 mg/kg of body weight or 0.1 mg/kg of body weight to 10 mg/kg of body weight. Alternatively, the FLT3 inhibitor may be administered in an amount of 0.01 mg/m² to 1000 mg/m² of body surface area, e.g., 3.7 mg/m² to 370 mg/m² of body surface area.

The HMA in the pharmaceutical composition according to a specific embodiment may be administered in an amount of 0.1 mg to 2000 mg, e.g., 1 mg to 1500 mg, 20 mg to 500 mg, or 1 mg to 150 mg. Alternatively, the HMA may be administered in an amount of 0.001 mg/kg of body weight to 400 mg/kg of body weight, e.g., 0.001 mg/kg of body weight to 100 mg/kg of body weight, or 0.2 mg/kg of body weight to 200 mg/kg of body weight. Alternatively, the HMA may be administered in a daily dose of 0.01 mg/kg of body weight to 300 mg/kg of body weight, e.g., in a daily dose of 0.2 mg/kg of body weight to 200 mg/kg of body weight or 0.017 mg/kg of body weight to 2.5 mg/kg of body weight. Alternatively, the HMA may be administered in an amount of 0.01 mg/m² to 1000 mg/m² of body surface area, e.g., 6 mg/m² to 92.5 mg/m² of body surface area or 7.4 mg/m² to 740 mg/m² of body surface area.

An amount of the combined two drugs to be administered to a patient may be determined by the attending diagnostician, as one skilled in the art, by the use of known techniques and by observing results obtained under analogous circumstances. In determining the effective amount or dose of the compound to be administered, a number of factors are considered by the attending diagnostician, including, but not limited to: species of a mammal; size, age, and general health thereof; specific neoplasm involved; degree or involvement or severity of the neoplasm; response of an individual patient; a particular compound administered; mode of administration; bioavailability characteristics of a preparation administered; a dose regimen selected; use of concomitant medication; and other relevant circumstances. For example, when orally administered, the daily dose may be about 0.001 mg/kg to about 100 mg/kg, for example, about 0.005 mg/kg to about 30 mg/kg, for example, about 0.01 mg/kg to about 10 mg/kg per a patient's body weight. When intravenously administered, the daily dose may be appropriately about 0.0001 mg/kg to about 10 mg/kg of a patient's body weight, the whole being administered in one or more divided doses per day. In addition, a transmucosal preparation is administered at a dose of about 0.001 mg/kg to about 100 mg/kg per body weight, and may be administered once a day or dividedly administered several times a day. For example, azacitidine may be administered in an amount of about 250 mg to about 500 mg per day.

As the HMA according to one specific embodiment, 4-amino-1-β-D-ribofuranosyl-1,3,5-triazin-2(1H)-one (ingredient name: azacitidine, trade name: Vidaza) may be administered in an amount of about 5 mg per 1 m² of a patient's body surface area to about 125 mg per 1/m² of a patient's body surface area, e.g., in an amount of about 50 mg/m² to about 100 mg/m², e.g., in an amount of about 75 mg/m². A recommended starting dose for a first treatment cycle, for all patients regardless of baseline hematology laboratory values, is 75 mg/m² of Vidaza by subcutaneous (SC) or intravenous (IV) injection, daily for 7 days. Patients may be premedicated for nausea and vomiting. Treatment cycles may be repeated every 4 weeks. The dose may be increased to 100 mg/m², when no beneficial effect is not observed after 2 treatment cycles and when no toxicity other than nausea and vomiting has occurred. It is recommended that patients be treated for a minimum of 4 cycles to 6 cycles. Complete or partial response may require additional treatment cycles.

A dosage of the pharmaceutical composition according to one specific embodiment, or a dosage or a therapeutically effective amount of the FLT3 inhibitor and HMA in the composition may vary within a wide tolerance dose, and may be determined in a manner known in the art. The dosage will be adjusted according to individual requirements of each particular case, including a patient to be treated as well as a specific compound to be administered, route of administration (oral administration, parenteral administration), and conditions to be treated.

The daily dose may be administered as a single dose or as divided doses, or, in the case of parenteral administration, may be given as a continuous infusion.

The FLT3 inhibitor and the HMA in the pharmaceutical composition according to one specific embodiment may be administered simultaneously, sequentially, or individually without a specific time limit. Here, such administration means providing two compounds in therapeutically effective levels in the body of the patient. The interval between administrations may be several seconds, several minutes, several hours, or a predetermined number of days, and may have a pause, if necessary.

As used herein, the term "composition" or "pharmaceutical composition" refers to a mixture comprising the compound disclosed herein and at least one, and optionally, more than one other pharmaceutically acceptable chemical ingredient, e.g., a pharmaceutically acceptable additive.

The pharmaceutical composition may further include any one or more pharmaceutically acceptable additives selected from the group consisting of excipients, binders, disintegrants, lubricants, and any combination thereof. The additive is any substance known to those skilled in the art to be useful in the preparation of formulations, and may be adjusted as needed, for example, according to a mode of administration of a drug.

Also disclosed herein is a pharmaceutical kit, wherein the pharmaceutical composition is administered simultaneously, sequentially, in reverse order, or individually.

Another aspect of the present disclosure provides a pharmaceutical combination for treating AML, the pharmaceutical combination comprising, as active ingredients, (a) the FLT3 inhibitor or any pharmaceutically acceptable salt or hydrate thereof, and (b) the HMA or any pharmaceutically acceptable salt or hydrate thereof,
wherein these active ingredients are administered simultaneously, sequentially, in reverse order, or individually.

Also disclosed herein is a method of treating AML, the method including administering the following active ingredients simultaneously, sequentially, in reverse order, or individually:
(a) a therapeutically effective amount of the FLT3 inhibitor or any pharmaceutically acceptable salt or hydrate thereof; and
(b) the HMA or any pharmaceutically acceptable salt or hydrate thereof.

Also disclosed herein is a method of treating AML, the method including simultaneously, sequentially, in reverse order, or individually:
(a) administering, to a patient, a therapeutically effective amount of the FLT3 inhibitor or any pharmaceutically acceptable salt or hydrate thereof; and
(b) administering, to the patient, the HMA or any pharmaceutically acceptable salt or hydrate thereof.

In one specific embodiment, at least one of (a) and (b) may be under the direction or control of a physician.

Also disclosed herein is a method of treating AML in a patient, the method including the following steps simultaneously, sequentially, in reverse order, or individually:
(a) prescribing a patient to self-administer a therapeutically effective amount of the FLT3 inhibitor or any pharmaceutically acceptable salt or hydrate thereof; and
(b) administering, to the patient, the HMA or any pharmaceutically acceptable salt or hydrate thereof.

Also disclosed herein is a pharmaceutical combination for treating AML, the pharmaceutical combination comprising, as active ingredients, the FLT3 inhibitor or any pharmaceutically acceptable salt or hydrate thereof, and the HMA or any pharmaceutically acceptable salt or hydrate thereof, wherein these two active ingredients are administered simultaneously, sequentially, or individually.

In one example the pharmaceutical combination comprises the FLT3 inhibitor or any pharmaceutically acceptable salt or hydrate thereof, and the HMA or any pharmaceutically acceptable salt or hydrate thereof. The FLT3 inhibitor and the HMA in the combination include salts or hydrates produced from these two ingredients. For example, the production of the salts may be partially or completely performed.

Also disclosed herein is a method of treating a subject suffering from AML using the composition comprising, as active ingredients, the FLT3 inhibitor or any pharmaceutically acceptable salt or hydrate thereof, and the HMA or any pharmaceutically acceptable salt or hydrate thereof. In this regard, the two active ingredients may be administered simultaneously, sequentially, in reverse order, or individually. The treatment method according to one specific embodiment provides a method of treating AML with FLT3 mutations using the composition.

In one specific embodiment, the AML includes mutant FLT3 polynucleotide-positive AML, FLT3 ITD-positive AML, or AML with FLT3 point mutations.

The combination therapy of the FLT3 inhibitor and the HMA has improved therapeutic effects, as compared with single administration of the FLT3 inhibitor or the HMA. The therapeutic effects by using the combination exhibit a synergistic effect, where the combined effect of two or more drugs is greater than the arithmetical sum of the individual effects.

As used herein, the term "therapeutically effective amount" is an amount of the compound that treats AML, when administered in combination to a subject, i.e., a patient. An amount demonstrated to be a therapeutically effective amount for a specific subject at a predetermined moment may not be effective for 100% of subjects similarly treated for the disease, even though such a dose would be considered as a therapeutically effective amount by a clinician. An amount of the compound corresponding to a therapeutically effective amount may depend on a specific type of cancer, a stage of the cancer, age of a patient being treated, and other factors. In general, therapeutically effective amounts of these compounds are well known in the art.

In addition, the therapeutically effective amount may be a combination amount to treat AML, although one or both of the FLT3 inhibitor and the HMA is/are administered in a sub-therapeutically effective amount or dose. The sub-therapeutically effective amount is an amount of the compound that, when administered alone to a patient, does not completely inhibit biological activity of an intended target over time.

The administration or use of the combination may be at therapeutically effective intervals. The therapeutically effective interval is a period of time beginning when one of the compounds is administered to a patient and ending at the limit of the other compound at which the benefits of combined administration of the two compounds are maintained. Thus, combined administration may be performed simultaneously, sequentially, or in any order.

The time period or cycle of combined administration may be a total of 1 week, 28 days, 1 month, 2 months, 3 months, or 4 months, or more. The individual drugs may be each administered daily for the entire duration or only a portion of the time period or cycle. For example, for a 28-day cycle, the FLT3 inhibitor or any pharmaceutically acceptable salt or hydrate thereof may be administered daily for the cycle, whereas the HMA or any pharmaceutically acceptable salt or hydrate thereof may be administered for a portion of the period, such as for 5 consecutive days, 7 consecutive days, or 10 consecutive days, in which 5, 7, and 10 consecutive days may be the first 5, 7, or 10 days of the period or cycle, respectively.

As used herein, the term "combination" or "pharmaceutical combination" refers to a product produced by mixing or combining two or more active ingredients, and includes both fixed and non-fixed combinations of the active ingredients. The term "fixed combination" means that active ingredients, e.g., the compound disclosed herein and one or more additional therapeutic agents, are simultaneously administered to a subject in the form of a single entity or dosage. The term "non-fixed combination" means that active ingredients, e.g., the compound disclosed herein and one or more additional therapeutic agents are administered to a subject as separate entities either simultaneously, concurrently or sequentially with no specific time limits, wherein such administration provides therapeutically effective levels of the active ingredients for the body of the subject. The latter may also be applied to a cocktail therapy, for example, administration of three or more active ingredients.

As used herein, the term "subject" encompasses mammals including humans and non-mammals. Examples of mammals include humans, chimpanzees, apes, monkeys, cattle, horses, sheep, goats, pigs; rabbits, dogs, cats, rats, mice, guinea pigs, etc., but are not limited thereto. Examples of non-mammals include birds, fish, etc., but are not limited thereto.

As used herein, the term "treating", "treat", "to be treated", or "treatment" include restraining, slowing, arresting, reducing, or reversing the progression or severity of an existing symptom, disease, condition, or disorder.

As used herein, the term "to" refers to a range including the numerical values described before and after the term "to" as a lower limit and an upper limit, respectively. It means the interval between the numerical values including the numerical values described before and after. The numerical value may be a range obtained by selecting and combining any number of upper and/or lower limits.

With regard to industrial applicability herein, utility of this combination therapy is exemplified by positive effects in one or more studies, including description of one or more parameters.

Hereinafter, the present disclosure will be described in more detail with reference to the following exemplary embodiments and experimental exemplary embodiments. However, these exemplary embodiments and experimental exemplary embodiments are only for better understanding of the present disclosure, and the scope of the present disclosure is not limited thereto in any sense.

### [Example 1]

### Evaluation of cell growth inhibitory activity under conditions of combination treatment with azacitidine

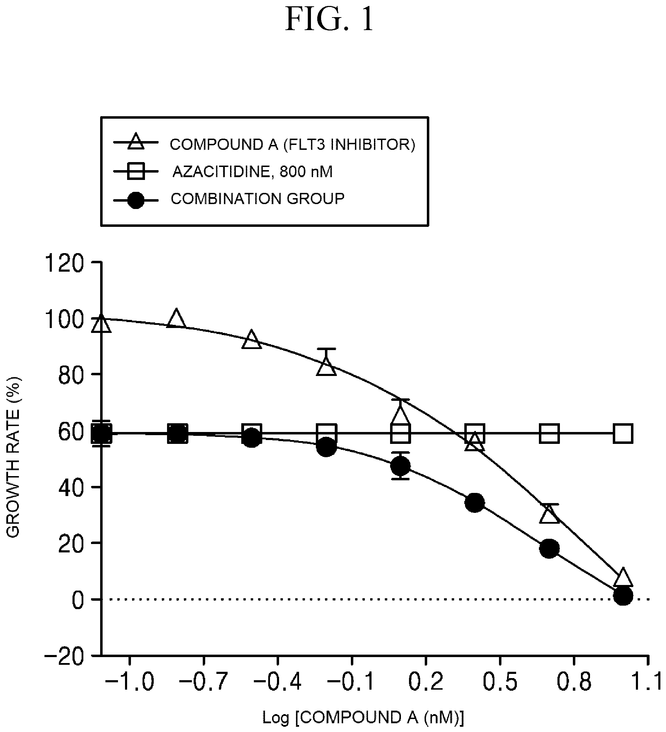

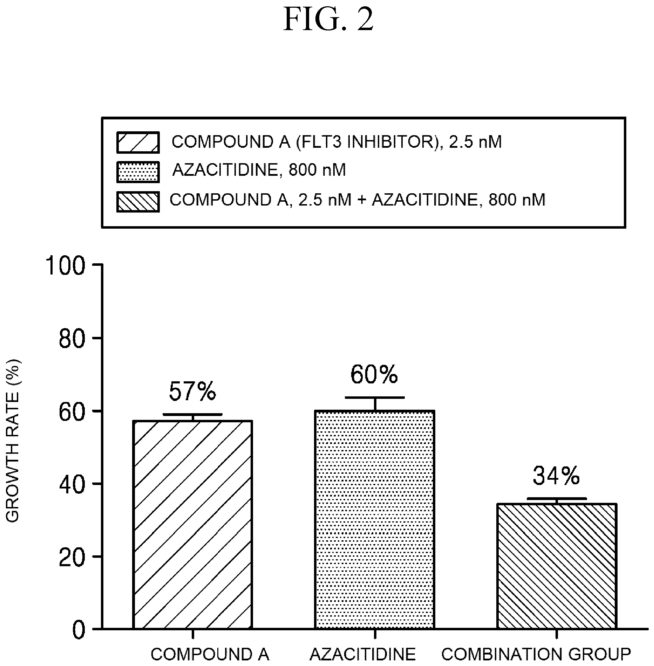

By examining growth inhibition of an MOLM-13 (DSMZ no. ACC 554) cell line which was treated with, in combination, an FLT3 inhibitor, 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine (hereinafter, Compound A) and a hypomethylating agent (HMA), 4-amino-1-β-D-ribofuranosyl-1,3,5-triazin-2(1H)-one (hereinafter, azacitidine), an effect of combined treatment of the two drugs was tested. The MOLM-13 cell line cultured in an RPMI 1640 culture medium (RPMI=Rosewell Parker Memory Institute) supplemented with 20% FBS was inoculated at a density of 2 × 10⁴ cells per well in a 96-well plate, and Compound A was subjected to 1/2 serial dilution at predetermined concentrations (e.g., 10 nM to 0.078 nM) using the same culture medium. Azacitidine was diluted using the culture medium at a concentration of 800 nM which is a concentration (GI₄₀) inhibiting MOLM-13 cell growth by about 40%, and then treated in combination with Compound A or treated alone, followed by incubation for 3 days. To measure cell viability, a CellTiter-Glo^{®}(CTG) assay was performed, and for result analysis, 50% cell growth inhibition (GI₅₀) was calculated using a GraphPad Prism software. The results are shown in Table 3 and FIGS. 1 and 2.

Table 3 below shows data for MOLM-13 cell growth inhibitory activity by treatment with Compound A alone or by treatment in combination.

**[Table 3]**

| Compound | GI₅₀(nM) |
|---|---|
| Compound A alone | 2.5 |
| Compound A + Azacitidine | 0.44 |

FIG. 1 shows inhibition of MOLM-13 cell growth by treatment with Compound A and azacitidine in combination. The Y-axis represents a cell growth rate (%) and the X-axis represents a logarithmic concentration of Compound A (a logarithmic value of a unit concentration in nM).

FIG. 2 shows inhibition of cell growth by treatment with 2.5 nM of Compound A, 800 nM of azacitidine, or combination of 2.5 nM of Compound A and 800 nM of azacitidine, wherein the Y-axis represents a cell growth rate (%) and the X-axis represents each experimental group.

As a result, as shown in Table 3 and FIGS. 1 and 2, combination treatment with Compound A and azacitidine exhibited excellent cell growth inhibitory effect, as compared with treatment with Compound A alone or azacitidine alone.

### [Example 2]

Mouse model xenografted with MOLM-13-Luc2 cell line

A comparison of Compound A and azacitidine or an efficacy test of combination thereof was conducted in a mouse model xenografted with a MOLM-13-Luc2 cell line which is a MOLM-13 cell line genetically modified to express luciferase.

The MOLM-13-Luc2 cell line was injected to a tail vein of NOG mouse at 5×10⁶ cells/0.1 mL/mouse, and allowed to grow.

Bioluminescence images of MOLM-13-Luc2 cells were measured using a Lumina III IVIS imaging system (PerkinElmer) and quantified using a Living image software (PerkinElmer). When measured, D-luciferin (D-luciferin) was intraperitoneally injected to mice, and imaging was were performed under anesthesia using isoflurane. Images were measured for a first group separation during the experiment, and then measured on a specific day according to the purpose of the test.

A control group orally received a DMSO/PEG400/DW (ratio=0.5/2/7.5, v/v) mixed solution once a day, and a Compound A group was orally administered once a day at a dose of 10 mg/kg/day. An azacitidine group which is a hypomethylating agent (HMA) group was intraperitoneally administered at a dose of 3 mg/kg/day twice a week (on day 1 and day 4 of the week). A combination group was orally administered with Compound A at a dose of 10 mg/kg/day once a day, and azacitidine was intraperitoneally administered at a dose of 3 mg/kg/day twice a week (on day 1 and day 4 every week). Each group received individual drugs until all subjects in each group died.

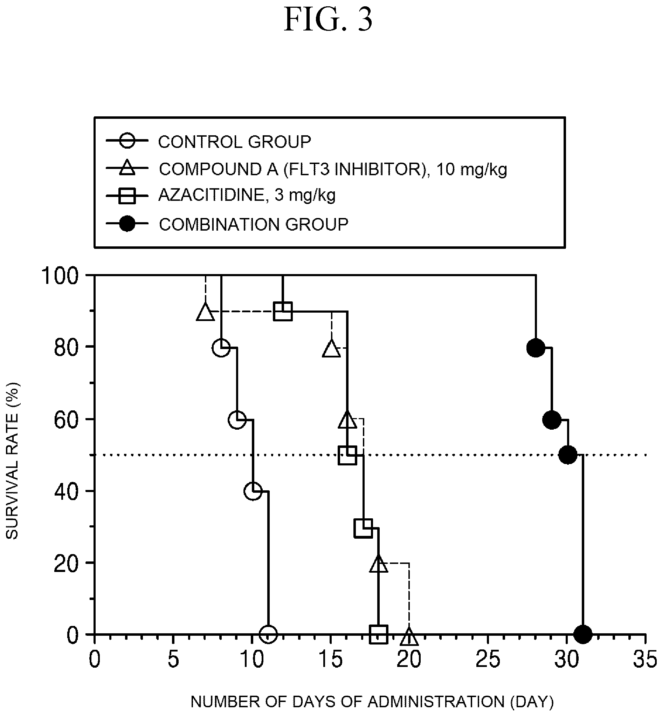

The experimental results are shown in FIG. 3. FIG. 3 shows anti-tumor effects when nude mice xenografted with the MOLM-13-Luc2 cell line were treated with Compound A (FLT3 inhibitor) and azacitidine in combination. The Y-axis represents a percentage (%) of surviving mice (survival rate) in each experimental group, and the X-axis represents the number of days of administration.

As shown in FIG. 3, the antitumor effects according to drug administration were examined by measuring the survival period, and as a result, a median survival period and an overall survival period in the combination group were 30 days and 31 days, respectively. In addition, as shown in FIG. 3, the results were higher than the median survival period (16 days) and the overall survival period (18 days) in the azacitidineadministered group, which is an HMA-administered group, and were higher than the median survival period (17 days) and the overall survival period (20 days) in the Compound A-administered group.

In addition, from the experimental results using a mouse efficacy model orthotopic transplanted with MOLM-13-Luc2 shown in FIG. 3, the combination group of FLT3 inhibitor and HMA showed the increased survival period and better antitumor efficacy, as compared with the group administered with Compound A alone which is an FLT3 inhibitor (single treatment with Compound A) or the group administered with HMA alone (single treatment with azacitidine).

Further, the above results indicate that combination of 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine which is an FLT3 inhibitor and 4-amino-1-β-D-ribofuranosyl-1,3,5-triazin-2(1H)-one (azacitidine) which is an HMA exhibits improved anti-tumor effects.

## Claims

1. A pharmaceutical composition for use in treating acute myeloid leukemia (AML), the pharmaceutical composition comprising an Fms-like tyrosine kinase (Fms-like tyrosine kinase-3: FLT3) inhibitor or a pharmaceutically acceptable salt or solvate thereof,
administered in combination with a hypomethylating agent (HMA) or a pharmaceutically acceptable salt or solvate thereof,
wherein the FLT3 inhibitor is 5-chloro-N-(3-cyclopropyl-5-(((3R,5S)-3,5-dimethylpiperazin-1-yl)methyl)phenyl)-4-(6-methyl-1H-indol-3-yl)pyrimidin-2-amine or a pharmaceutically acceptable salt, solvate, stereoisomer or tautomer thereof,
and wherein the HMA is any one selected from 4-amino-1-β-D-ribofuranosyl-1,3,5-triazin-2(1H)-one (azacitidine), 4-amino-1-((2R,4R,5R)-4-hydroxy-5-(hydroxymethyl)tetrahydrofuran-2-yl)-1,3,5-triazin-2(1H)-one (decitabine), and a pharmaceutically acceptable salt or hydrate thereof.

2. The pharmaceutical composition for use in treating AML as in claim 1, wherein the HMA is 4-amino-1-β-D-ribofuranosyl-1,3,5-triazin-2(1H)-one.

3. The pharmaceutical composition for use in treating AML as in claim 1, wherein the HMA is 4-amino-1-((2R,4R,5R)-4-hydroxy-5-(hydroxymethyl)tetrahydrofuran-2-yl)-1,3,5-triazin-2(1H)-one.

4. The pharmaceutical composition for use in treating AML as in claim 1, wherein the AML is AML with FLT3 mutations.

5. The pharmaceutical composition for use in treating AML as in claim 1, wherein the AML is mutant FLT3 polynucleotide-positive AML, FLT3 internal tandem duplication (ITD)-positive AML, or AML with FLT3 point mutations.

6. The pharmaceutical composition for use in treating AML as in claim 1, wherein the AML has a mutation in a tyrosine kinase domain (TKD) of an FLT3 amino acid sequence (FLT3-TKD).

7. The pharmaceutical composition for use in treating AML as in claim 6, wherein the FLT3-TKD mutation further comprises internal tandem duplication (ITD).

8. The pharmaceutical composition for use in treating AML as in claim 6, wherein the FLT3-TKD mutation comprises any one selected from FLT3(D835Y), FLT3(F691L), FLT3(F691L/D835Y), FLT3(ITD/D835Y), FLT3(ITD/F691L), and a combination thereof.

## Patentansprüche

1. Pharmazeutische Zusammensetzung zur Verwendung bei der Behandlung von akuter myeloischer Leukämie (AML), wobei die pharmazeutische Zusammensetzung einen Inhibitor von FMS-ähnlicher Tyrosinkinase (FMS-ähnlicher Tyrosinkinase-3: FLT3) oder ein pharmazeutisch unbedenkliches Salz oder Solvat davon umfasst,
der in Kombination mit einem Hypomethylierungsmittel (HMA) oder einem pharmazeutisch unbedenklichen Salz oder Solvat davon verabreicht wird,
wobei es sich bei dem FLT3-Inhibitor um 5-Chlor-N-(3-cyclopropyl-5-(((3R,5S)-3,5-dimethylpiperazin-1-yl)methyl)phenyl)-4-(6-methyl-1H-indol-3-yl)pyrimidin-2-amin oder ein pharmazeutisch unbedenkliches Salz, Solvat, Stereoisomer oder Tautomer davon handelt
und wobei es sich bei dem HMA um eines handelt, das aus 4-Amino-1-β-D-ribofuranosyl-1,3,5-triazin-2(1H)-on (Azacitidin), 4-Amino-1-((2R,4R,5R)-4-hydroxy-5-(hydroxymethyl)tetrahydrofuran-2-yl)-1,3,5-triazin-2(1H)-on (Decitabin) oder einem pharmazeutisch unbedenklichen Salz oder Hydrat davon ausgewählt ist.

2. Pharmazeutische Zusammensetzung zur Verwendung bei der Behandlung von AML nach Anspruch 1, wobei es sich bei dem HMA um 4-Amino-1-β-D-ribofuranosyl-1,3,5-thazin-2(1H)-on handelt.

3. Pharmazeutische Zusammensetzung zur Verwendung bei der Behandlung von AML nach Anspruch 1, wobei es sich bei dem HMA um 4-Amino-1-((2R,4R,5R)-4-hydroxy-5-(hydroxymethyl)tetrahydrofuran-2-yl)-1,3,5-triazin-2(1H)-on handelt.

4. Pharmazeutische Zusammensetzung zur Verwendung bei der Behandlung von AML nach Anspruch 1, wobei es sich bei der AML um AML mit FLT3-Mutationen handelt.

5. Pharmazeutische Zusammensetzung zur Verwendung bei der Behandlung von AML nach Anspruch 1, wobei es sich bei der AML um mutierte FLT3-Polynukleotid-positive AML, FLT3-interne-Tandemduplikation(ITD)-positive AML oder AML mit FLT3-Punktmutationen handelt.

6. Pharmazeutische Zusammensetzung zur Verwendung bei der Behandlung von AML nach Anspruch 1, wobei die AML eine Mutation in einer Tyrosinkinasedomäne (TKD) einer FLT3-Aminosäuresequenz (FLT3-TKD) aufweist.

7. Pharmazeutische Zusammensetzung zur Verwendung bei der Behandlung von AML nach Anspruch 6, wobei die FLT3-TKD-Mutation ferner interne Tandemduplikation (ITD) umfasst.

8. Pharmazeutische Zusammensetzung zur Verwendung bei der Behandlung von AML nach Anspruch 6, wobei die FLT3-TKD-Mutation eine beliebige umfasst, die aus FLT3(D835Y), FLT3(F691L), FLT3(F691L/D835Y), FLT3(ITD/D835Y), FLT3(ITD/F691L) und einer Kombination davon ausgewählt ist.

## Revendications

1. Composition pharmaceutique destinée à être utilisée dans le traitement de la leucémie myéloïde aiguë (AML), la composition pharmaceutique comprenant un inhibiteur de tyrosine kinase de type Fms (tyrosine kinase de type Fms 3 : FLT3) ou un sel ou solvate pharmaceutiquement acceptable correspondant,
administrée en combinaison avec un agent hypométhylant (HMA) ou un sel ou solvate pharmaceutiquement acceptable correspondant,
dans laquelle l'inhibiteur de FLT3 est la 5-chloro-N-(3-cyclopropyl-5-(((3R,5S)-3,5-diméthylpipérazin-1-yl)méthyl)phényl)-4-(6-méthyl-1H-indol-3-yl)pyrimidin-2-amine ou un sel, un solvate, un stéréoisomère ou une forme tautomère pharmaceutiquement acceptable correspondant(e),
et dans laquelle l'HMA est l'une quelconque choisi parmi 4-amino-1-β-D-ribofuranosyl-1,3,5-triazin-2(1H)-one (azacitidine), 4-amino-1-((2R,4R,5R)-4-hydroxy-5-(hydroxyméthyl)tétrahydrofuran-2-yl)-1,3,5-triazin-2(1H)-one (décitabine), et un sel ou hydrate pharmaceutiquement acceptable correspondant.

2. Composition pharmaceutique destinée à être utilisée dans le traitement de l'AML selon la revendication 1, dans laquelle l'HMA est la 4-amino-1-β-D-ribofuranosyl-1,3,5-thazin-2(1H)-one.

3. Composition pharmaceutique destinée à être utilisée dans le traitement de l'AML selon la revendication 1, dans laquelle la HMA est la 4-amino-1-((2R,4R,5R)-4-hydroxy-5-(hydroxyméthyl)tétrahydrofuran-2-yl)-1,3,5-triazin-2(1H)-one.

4. Composition pharmaceutique destinée à être utilisée dans le traitement de l'AML selon la revendication 1, dans laquelle l'AML est l'AML comportant des mutations de FLT3.

5. Composition pharmaceutique destinée à être utilisée dans le traitement de l'AML selon la revendication 1, dans laquelle l'AML est une AML positive à un polynucléotide de FLT3 mutant, une AML positive à une duplication en tandem interne (ITD) de FLT3 ou une AML comportant des mutations ponctuelles de FLT3.

6. Composition pharmaceutique destinée à être utilisée dans le traitement de l'AML selon la revendication 1, dans laquelle l'AML a une mutation dans un domaine de tyrosine kinase (TKD) d'une séquence d'acides aminés de FLT3 (FLT3-TKD).

7. Composition pharmaceutique destinée à être utilisée dans le traitement de l'AML selon la revendication 6, dans laquelle la mutation de FLT3-TKD comprend en outre une duplication en tandem interne (ITD).

8. Composition pharmaceutique destinée à être utilisée dans le traitement de l'AML selon la revendication 6, dans laquelle la mutation de FLT3-TKD comprend l'une quelconque choisie parmi FLT3(D835Y), FLT3(F691L), FLT3(F691L/D835Y), FLT3(ITD/D835Y), FLT3(ITD/F691L), et une combinaison de celles-ci.
